(19) Europäisches Patentamt
European Patent Office
Office européen des brevets

(11) **EP 1 335 991 B1**

(12) **EUROPÄISCHE PATENTSCHRIFT**

(45) Veröffentlichungstag und Bekanntmachung des Hinweises auf die Patenterteilung:
**04.10.2006 Patentblatt 2006/40**

(21) Anmeldenummer: **01995659.8**

(22) Anmeldetag: **22.11.2001**

(51) Int Cl.:
**C12Q 1/68** $^{(2006.01)}$

(86) Internationale Anmeldenummer:
**PCT/EP2001/013625**

(87) Internationale Veröffentlichungsnummer:
**WO 2002/042492 (30.05.2002 Gazette 2002/22)**

(54) **VERFAHREN ZUM NACHWEIS VON PROTOZOEN DER GATTUNG NAEGLERIA**

METHOD FOR DETECTING PROTOZOAE OF THE GENUS NAEGLERIA

PROCEDE POUR DETECTER DES PROTOZOAIRES DU GENRE NAEGLERIA

(84) Benannte Vertragsstaaten:
**AT BE CH CY DE DK ES FI FR GB GR IE IT LI LU MC NL PT SE TR**

(30) Priorität: **22.11.2000 DE 10057841**

(43) Veröffentlichungstag der Anmeldung:
**20.08.2003 Patentblatt 2003/34**

(73) Patentinhaber: **Vermicon AG**
**80992 München (DE)**

(72) Erfinder:
• **SNAIDR, Jiri**
**80992 München (DE)**
• **TREBESIUS, Karlheinz**
**80992 München (DE)**

(74) Vertreter: **Bohmann, Armin K.**
**Bohmann & Loosen,**
**Anwaltssozietät,**
**Sonnenstrasse 8**
**80331 München (DE)**

(56) Entgegenhaltungen:
• **KIVINGTON S ET AL : "Identification and epidemiological typing of naegleri fowleri with DNA probes" APPLIED AND ENVIRONMENTAL MICROBIOLOGY, Bd. 61, Nr. 6, Juni 1995 (1995-06), Seiten 2071-78, XP002204233 in der Anmeldung erwähnt**
• **P¹LANDAKIS M ET AL: "Genetic variation in free-living amoeba Naegleria fowleri " APPLIED AND ENVIRONMENTAL MICROBIOLOGY, Bd. 64, Nr. 8, August 1998 (1998-08), Seiten 2977-81, XP002204234**
• **TREBESIUS K ET AL: "Development of rRNA targeted PCR and in situ hybridization with fluorescently labelled oligonucleotides for the detection of Yersinia species" JOURNAL OF CLINICAL MICROBIOLOGY, Bd. 36, Nr. 9, September 1998 (1998-09), Seiten 2557-64, XP002204235**

**Beschreibung**

Die Erfindung betrifft ein Verfahren zum schnellen und spezifischen Nachweis von Protozoen der Gattung *Naegleria* und insbesondere der Art *Naegleria fowleri*. Weiter betrifft die Erfindung spezifische Oligonukleotidsonden, die im Rahmen des Nachweisverfahrens eingesetzt werden, sowie Kits, die diese Oligonukleotidsonden enthalten.

Naeglerien sind kleine, freilebende Amöbenflagellaten, die weltweit verbreitet sind, und vor allem in Wasserproben gefunden werden. Von den bis heute bekannten Naeglerienarten ist nur *Naegleria fowleri* sicher pathogen. *Naegleria fowleri* kommt in drei unterschiedlichen Formen vor: Zyste, Amöbe und Flagellat. Im warmen Wasser kann der Einzeller sehr schnell vom Amöbenstadium in das Flagellatenstadium wechseln, was ihm eine recht schnelle Fortbewegung ermöglicht. Die Infektion mit diesen freilebenden Amöben erfolgt wahrscheinlich beim Baden und Tauchen in Süßwassergewässern (insbesondere in ruhigen, warmen Teichen oder Seen, bisweilen auch in schlecht gepflegten Whirlpools). Die Amöben dringen nasal ein, gelangen entlang des Nervus olfactorius in das Gehirn und produzieren dort ein Toxin, welches das Gehirn zersetzt. Der Organismus bildet keine Zysten im menschlichen Organismus. Diese Krankheit wird als Amöben-Meningoenzephalitis (PAM; Primary Amebic Meningoencephalitis) bezeichnet. Sie ist rasch voranschreitend und endet fast immer tödlich. PAM tritt vorwiegend bei Kindern oder jüngeren Erwachsenen aus völliger Gesundheit heraus auf. Plötzlicher massiver Krankheitsbeginn mit Fieber, Übelkeit, Erbrechen, Kopfschmerz und Nakkensteifigkeit. Es kommt zu einer raschen Progredienz unter dem Bild einer pyogenen Meningoenzephalitis, die Patienten werden komatös und versterben meist innerhalb von 1 Woche, ohne dass neurologische Herdsymptome auffällig werden. Die Diagnose findet meist erst postmortem nach einer Gehirnautopsie statt. Die einzigen momentan bekannten Möglichkeiten zur Behandlung sind die hochdosierte systemische und intrathekale Gabe von Amphotericin B und Miconazol kombiniert mit oraler Verabreichung von Rifampicin und die Gabe von Amphotericin B in Verbindung mit Metronidazol.

Für eine rechtzeitige Behandlung ist aber die sehr schnelle Diagnose der infektiösen Keime die Voraussetzung. Möglicherweise besitzt auch *Naegleria australiensis* pathogene Eigenschaften, die aber im Vergleich zu *Naegleria fowleri* geringer sind.

Die Untersuchung von menschlichen Proben, von Badegewässern, Swimming-Pools, Hallenbädern und Whirlpools hinsichtlich des Vorhandenseins von *Naegleria*-Arten wäre somit äußerst wichtig und im Sinne einer präventiven Gesundheitsstrategie von großem öffentlichen Interesse. Es bedarf daher sehr schneller und präziser Identifizierungsmethoden, um Naeglerienarten nachzuweisen und pathogene von nicht-pathogenen Arten unterscheiden zu können.

Es gibt heutzutage mehrere Ansätze *Naegleria fowleri* zu identifizieren. Diese beinhalten serologische Tests mit spezifischen monoklonalen Antikörpern (Visvesvara *et al.* (1987) Production of monoclonal antibodies to *Naegleria fowleri*, agent of primary amebic meningoencephalitis. J. Clin. Microbiol. 25:1629-1634), elektrophoretischen Profilen von Isoenzymen (De Jonckheere (1981) *Naegleria australiensis* sp. nov., another pathogenic *Naegleria* from water. Protistologica XVII:423-429), die Charakterisierung von DNA-Restriktionsfragmentlängenpolymorphismen (RFLPs, restriction fragment length polymorphisms) (McLaughlin *et al.* (1988) Restriction fragment length polymorphisms of the DNA of selected *Naegleria* and *Acanthamoeba* amoebae. J. Clin. Microbiol. 26:1655-1658) oder mittels PCR (polymerase chain reaction, Polymerase-Kettenreaktion) (Sparagano (1993) Differentiation of *Naegleria fowleri* and other naegleriae by polymerase chain reaction and hybridization methods. FEMS. Microbiol. Lett. 110:325-330). Aber diese Methoden sind sehr aufwendig, zeitintensiv und erfordern eine hohe Konzentration an Amöben. Überdies zeigen sie oft deutliche Unspezifitäten und sind daher für eine robuste, hochspezifischen und schnelle Analytik nicht geeignet. Eine Möglichkeit stellen auch DNA-Sonden zum speziesspezifischen Nachweis von Naeglerien nach Kultivierung und mittels Dot-Blot-Hybridisierung dar (Kilvington *et al.* (1995) Identification and epidemiological typing *of Naegleria fowleri* with DNA probes. Appl. Env. Microb. 61:2071-2078). Abgesehen davon, dass hier ein zeitaufwendiger Kultivierungsschritt notwendig ist, gefolgt von einer Nukleinsäurehybridisierung, können die im Stand der Technik beschriebenen Sonden schon aufgrund ihrer Länge von mehreren Hundert Basen in der Regel nicht für eine in situ- bzw. Ganzzellhybridisierung eingesetzt werden.

Aufgabe der vorliegenden Erfindung ist es, Sondensequenzen und ein Verfahren zur Verfügung zu stellen, mit deren Hilfe Protozoen der Gattung *Naegleria* schnell und spezifisch nachgewiesen und, falls erforderlich oder erwünscht, visualisiert werden können.

Erfindungsgemäß wird diese Aufgabe durch die Bereitstellung der folgenden Nukleinsäuresondenmoleküle gelöst:

a) Oligonukleotidmoleküle, die alle Arten der Gattung *Naegleria* (also *N. australiensis, N. italica, N. jamiesoni, N. andersoni, N. lovanensis, N. fowleri, N. gruberi, N. clarki* und *N. minor*) nachweisen:

NAEG1:     5'-ACC-ATA-GCG-CTC-GCT-GGT-3'

NAEG2:     5'-GTG-GCC-CAC-GAC-AGC-TTT-3'

b) Oligonukleotidmoleküle, die spezifisch die Art *Naegleria fowleri* nachweisen:

NFOW1:   5'-GGT-CGA-TGC-CCA-GCT-CCC-3'

NFOW2:   5'-GTC-AAA-GCC-TTG-TTT-GTC-3'.

Gegenstand der Erfindung sind auch Abwandlungen der Oligonukleotide NAEG1, NAEG2, NFOW1 und NFOW2, die trotz der Abweichungen in der Sequenz und/oder Länge eine spezifische Hybridisierung mit Nukleinsäuresequenzen von *Naegleria*-Arten zeigen.

Hierunter fallen insbesondere

a) Nukleinsäuremoleküle, die (i) mit den Oligonukleotidsequenzen NAEG1, NAEG2, NFOW1 oder NFOW2 in mindestens 60 %, 65 %, bevorzugt in mindestens 70 %, 75 %, bevorzugter in mindestens 80 %, 84 %, 87 % und besonders bevorzugt in mindestens 90 %, 94 %, 97 % der Basen übereinstimmen (wobei der Sequenzbereich des Nukleinsäuremolekül zu betrachten ist, der dem Sequenzbereich von NAEG1, NAEG2, NFOW1 und NFOW2 entspricht, und nicht etwa die gesamte Sequenz eines Nukleinsäuremoleküls, das u.U. im Vergleich zu NAEG1, NAEG2, NFOW1 und NFOW2 um eine bis zahlreiche Basen verlängert ist) oder (ii) sich von NAEG1, NAEG2, NFOW1 oder NFOW durch mindestens eine Deletion und/oder Addition unterscheiden und die eine spezifische Hybridisierung mit Nukleinsäuresequenzen von Naeglerien-Arten ermöglichen. Dabei bedeutet "spezifische Hybridisierung", dass unter den hier beschriebenen oder den dem Durchschnittsfachmann im Zusammenhang mit in situ-Hybridisierungstechniken bekannten Hybridisierungsbedingungen nur die ribosomale RNA der Ziel-Organismen (also z.B. von *N. fowleri* bei dem Oligonukleotid NFOW1), nicht aber die rRNA von Nicht-Ziel-Organismen (z.B. von *N. lovaniensis* bei der Sonde NFOW1) an das Oligonukleotid bindet.

b) Nukleinsäuremoleküle, die zu den unter a) genannten Nukleinsäuremolekülen oder den Sonden NAEG1, NAEG2, NFOW1 oder NFOW2 komplementär sind oder mit den unter a) genannten Nukleinsäuremolekülen oder den Sonden NAEG1, NAEG2, NFOW1 oder NFOW2 spezifisch hybridisieren;

c) Nukleinsäuremoleküle, die die Oligonukleotidsequenzen NAEG1, NAEG2, NFOW1, NFOW2 oder die Sequenz eines Nukleinsäuremoleküls nach a) oder b) umfassen, also zusätzlich zu den genannten Sequenzen bzw. deren Abwandlungen nach a) oder b) mindestens ein weiteres Nukleotid aufweisen, und eine spezifische Hybridisierung mit Nukleinsäuresequenzen von Naeglerien-Arten ermöglichen.

Der Grad der Sequenzidentität eines Nukleinsäuremoleküls mit den Sonden NAEG1, NAEG2, NFOW1 und NFOW2 kann mit üblichen Algorithmen bestimmt werden. Geeignet ist hier beispielsweise das Programm zur Bestimmung der Sequenzidentität, das unter http://www.ncbi.nlm.nih.gov/BLAST (auf dieser Seite z.B. der Link "Standard nucleotide-nucleotide BLAST [blastn]") zugänglich ist.

Die erfindungsgemäßen Nukleinsäuremoleküle, die als Sonden im Rahmen des Nachweises von Naeglerienarten eingesetzt werden können, umfassen synthetisch hergestellte Sondenmoleküle wie auch rekombinant hergestellte Sonden, die die oben genannten Sondensequenzen aufweisen. Zudem können an den nicht diskriminatorischen Positionen auch Nukleotidanaloga wie Inosin u.ä. die eigentlichen Nukleotide ersetzen. Weiterhin können die Sondenmoleküle auch mittels Nukleotidanaloga, wie PNA (peptide nucleic acids) u.ä. hergestellt werden.

Die genannten Oligonukleotidmoleküle und erfindungsgemäßen Abwandlungen dieser Moleküle werden gemäß der Erfindung in einem Verfahren zum Nachweis von Mikroorganismen in einer Probe mittels einer Nukleinsäuresonde eingesetzt, wobei das Verfahren folgende Schritte umfasst:

a) Fixieren der in der Probe enthaltenen *Naegleria*-Zellen

b) Inkubieren der fixierten Zellen mit mindestens einem der erfindungsgemäßen Nukleinsäuresondenmolekülen, insbesondere mit einem Nukleinsäuresondenmolekül ausgewählt aus der Gruppe, bestehend aus den Molekülen:

5'-ACC-ATA-GCG-CTC-GCT-GGT-3' ,
5'-GTG-GCC-CAC-GAC-AGC-TTT-3' ,
5'-GGT-CGA-TGC-CCA-GCT-CCC-3' und
5'-GTC-AAA-GCC-TTG-TTT-GTC-3',

um eine Hybridisierung herbeizuführen,

c) Entfernen nicht hybridisierter Nukleinsäuresondenmoleküle;

d) Detektieren und, gegebenenfalls Quantifizieren und Visualisieren, der *Naegleria*-Zellen mit hybridisierten Nukleinsäuresondenmolekülen.

Im Rahmen der vorliegenden Erfindung wird unter "Fixieren" der Zellen eine Behandlung verstanden, mit der die Zellhülle der Protozoen für Nukleinsäuresonden durchlässig gemacht wird. Die Nukleinsäuresonden, die aus einem Oligonukleotid und einem daran gebundenen Marker bestehen, können dann die Zellhülle penetrieren und sich an die der Nukleinsäuresonde entsprechenden Zielsequenz im Zellinneren binden. Die Bindung ist als Ausbildung von Wasserstoffbrücken zwischen komplementären Nukleinsäurestücken zu verstehen. Bei der Hülle kann es sich um eine Lipidhülle handeln, die einen Virus umgibt, um die Zellwand von Bakterien oder um die Zellmembran eines einzelligen Tierchens. Zur Fixierung wird üblicherweise eine niederprozentige Paraformaldehydlösung oder eine verdünnte Formaldehydlösung verwendet. Kann die Zellwand mit diesen Maßnahmen nicht von den Nukleinsäuresonden penetriert werden, so sind dem Fachmann ausreichend weitere Maßnahmen bekannt, die zu demselben Ergebnis führen. Dazu zählen beispielsweise Ethanol, Methanol, Mischungen dieser Alkohole, enzymatische Behandlungen oder ähnliches.

Bei der Nukleinsäuresonde im Sinne der Erfindung kann es sich um eine DNA- oder RNA-Sonde handeln, die in der Regel zwischen 12 und 1000 Nukleotide, bevorzugt zwischen 12 und 500, bevorzugter zwischen 12 und 200, besonders bevorzugt zwischen 12 und 50 und zwischen 15 und 40, und am meisten bevorzugt zwischen 17 und 25 Nukleotide umfassen wird. Die Auswahl der Nukleinsäuresonden geschieht nach den Gesichtspunkten, ob eine komplementäre Sequenz in dem nachzuweisenden Mikroorganismus vorliegt. Durch diese Auswahl einer definierten Sequenz, kann dadurch eine Bakterienart, eine Bakteriengattung oder eine ganze Bakteriengruppe erfaßt werden. Komplementarität sollte bei einer Sonde von 15 Nukleotiden über 100% der Sequenz gegeben sein. Bei Oligonukleotiden mit mehr als 15 Nukleotiden sind ein bis mehrere Fehlpaarungsstellen erlaubt. Durch das Einhalten von stringenten Hybridisierungsbedingungen wird gewährleistet, dass das Nukleinsäuresondenmolekül auch tatsächlich mit der Zielsequenz hybridisiert. Moderate Bedingungen im Sinne der Erfindung sind z.B. 0% Formamid in einem Hybridisierungspuffer wie er in Beispiel 1 beschrieben ist. Stringente Bedingungen im Sinne der Erfindung sind beispielsweise 20-80% Formamid im Hybridisierungspuffer.

Die Nukleinsäuresonde kann dabei komplementär zu einer chromosomalen oder episomalen DNA sein, aber auch zu einer mRNA oder rRNA des nachzuweisenden Mikroorganismus. Im Rahmen der vorliegenden Erfindung ist die Nukleinsäuresonde bevorzugt komplemenatär zur 18S RNA der nachzuweisenden *Naeglaria*-Arten. Von Vorteil ist es eine Nukleinsäuresonde zu wählen, die zu einem Bereich komplementär ist, der in einer Kopiezahl von mehr als 1 im nachzuweisenden Mikroorganismus vorliegt. Die nachzuweisende Sequenz liegt bevorzugt 500 - 100.000 mal pro Zelle vor, besonders bevorzugt 1.000 - 50.000 mal. Aus diesem Grunde wird bevorzugt die rRNA als Zielstelle verwendet, da die Ribosomen in der Zelle als Orte der Proteinbiosynthese vieltausendfach in jeder aktiven Zelle vorliegen.

Erfindungsgemäß wird die Nukleinsäuresonde mit dem im obengenannten Sinne fixierten Mikroorganismus inkubiert, um so ein Eindringen der Nukleinsäuresondenmoleküle in den Mikroorganismus und die Hybridisierung von Nukleinsäuresondenmolekülen mit den Nukleinsäuren des Mikroorganismus zu erlauben. Anschließend werden die nicht-hybridisierten Nukleinsäuresondenmoleküle durch übliche Waschschritte entfernt. Die spezifisch hybridisierten Nukleinsäuresondenmoleküle können anschließend in den jeweiligen Zellen detektiert werden. Voraussetzung hierfür ist, dass die Nukleinsäuresonde nachweisbar ist, z.B. dadurch, dass das Sondenmolekül durch kovalente Bindung mit einen Marker verknüpft ist. Als detektierbare Marker werden fluoreszierende Gruppen wie z.B. CY2 (erhältlich von Amersham Life Sciences, Inc., Arlington Heights, USA), CY3 (ebenfalls erhältlich von Amersham Life Sciences), CY5 (ebenfalls zu beziehen von Amersham Life Sciences), FITC (Molecular Probes Inc., Eugene, USA), FLUOS (erhältlich von Roche Diagnostics GmbH, Mannheim, Deutschland), TRITC (erhältlich von Molecular Probes Inc. Eugene, USA) oder FLUOS-PRIME verwendet, die dem Fachmann alle wohlbekannt sind. Auch chemische Marker, radioaktive Marker oder enzymatische Marker wie MeerrettichPeroxidase, saure Phosphatase, alkalische Phosphatase, Peroxidase, können verwendet werden. Für jedes dieser Enzyme ist eine Reihe von Chromogenen bekannt, die anstelle des natürlichen Substrates umgesetzt werden können, und entweder zu farbigen oder zu fluoreszierenden Produkten umgesetzt werden können. Beispiele für solche Chromogene sind in der nachfolgenden Tabelle angegeben:

Tabelle

| Enzyme | Chromogen |
| --- | --- |
| 1. Alkalische Phosphatase und saure Phosphatase | 4-Methylumbelliferylphosphat (*), Bis(4-Methyiumbelliferylphosphat), (*) 3-O-Methylfluoreszein, Flavon-3-Diphosphattriammoniumsalz (*), p-Nitrophenylphosphatdinatriumsalz |

(fortgesetzt)

| Enzyme | Chromogen |
| --- | --- |
| 2. Peroxidase | Tyraminhydrochlorid (*), 3-(p-Hydroxyphenyl)-Propionsäure (*), p-Hydroxyphenethylalkohol(*), 2,2'-Azino-di-3-ethylbenzothiazolin sulfonsäure (ABTS), ortho-Phenylendiamindihydrochlorid, o-Dianisidin, 5-Aminosalicylsäure, p-Ucresol (*), 3,3'-dimethyloxybenzidin, 3-Methyl-2-benzothiazolinhydrazon, Tetramethylbenzidin |
| 3. Meerrettichperoxidase | $H_2O_2$ + Diammoniumbenzidin $H_2O_2$ + Tetramethylbenzidin |
| 4. β-D-Galaktosidase | o-Nitrophenyl-β-D-galaktopyranosid, 4-Methylumbelliferyl-β-D-galaktosid |
| 5. Glukoseoxidase | ABTS, Glukose und Thiazolylblau |

\* Fluoreszenz

Schließlich ist es möglich, die Nukleinsäuresondenmoleküle so zu gestalten, dass an ihrem 5'- oder 3'-Ende eine weitere zur Hybridisierung geeignete Nukleinsäuresequenz vorhanden ist. Diese Nukleinsäuresequenz umfasst wiederum ca. 15 bis 1.000, bevorzugt 15 - 50 Nukleotide. Dieser zweite Nukleinsäurebereich kann wiederum von einer Oligonukleotidsonde erkannt werden, die durch eines der oben erwähnten Mittel nachweisbar ist.

Eine weitere Möglichkeit besteht in der Kopplung der nachweisbaren Nukleinsäuresondenmoleküle mit einem Hapten, das anschließend mit einem das Hapten erkennenden Antikörper in Kontakt gebracht werden kann. Als Beispiel für solch ein Hapten kann Digoxigenin angeführt werden. Dem Fachmann sind über die angegebenen Beispiele auch noch weitere wohlbekannt.

Die Durchführung der Hybridisierung geschieht standardmäßig auf Objektträger, auf Filtern, auf einer Mikrotiterplatte oder in einem Reaktionsgefäß. Die Auswertung ist abhängig von der Art der Markierung der verwendeten Sonde möglich mit einem Lichtmikroskop, Epifluoreszenzmikroskop, Chemoluminometer, Fluorometer, Durchflußzytometer u.a.

Die erfindungsgemäßen Sondenmoleküle können im Rahmen des Nachweisverfahrens mit verschiedenen Hybridisierungslösungen eingesetzt werden. Verschiedene organische Lösungsmittel können in Konzentrationen von 0 % bis 80 % eingesetzt werden. Formamid wird beispielsweise vorzugsweise in einer Konzentration von 20 % bis 60 %, besonders bevorzugt in einer Konzentration von 20 % im Hybridisierungspuffer eingesetzt. Zudem ist ein Salz, vorzugsweise Natriumchlorid, in einer Konzentration von 0,1 mol/l bis 1,5 mol/l, bevorzugt von 0,5 mol/l bis 1,0 mol/l und bevorzugter von 0,7 mol/l bis 0,9 mol/l und am meisten bevorzugt von 0,9 mol/l im Hybridisierungspuffer enthalten. Zum Abpuffern des Hybridisierunaspuffers können verschiedene Verbindungen wie Tris-HCl, NatriumCitrat, PIPES oder HEPES-Puffer verwendet werden, die im Bereich von 0,01 mol/l bis 0,1 mol/l, bevorzugt von 0,01 mol/l bis 0,08 mol/l und besonders bevorzugt von 0,02 mol/l eingesetzt werden. Der pH-Wert liegt in der Regel im Bereich von 6,0 bis 9,0, bevorzugt von 7,0 bis 8,0. Bevorzugt weist der Hybridisierungspuffer 0,02 mol/l Tris-HCl, pH 8,0 auf.

Zudem sind meist Detergenzien, wie Triton X oder Natrium-Dodecylsulphat (SDS) in einer Konzentration von 0,001 % bis 0,2 %, bevorzugt von 0,05 % bis 0,1 % enthalten. Hier enthält ein besonders bevorzugter Hybridisierungspuffer 0,01 % SDS.

Weitere Zusatzstoffe können bei verschiedenen Problemstellungen zum Einsatz kommen, wie unmarkierte Nukleinsäurefragmente (z.B. fragmentierte Lachsspermien-DNA, unmarkierte Oligonukleotide u.ä.) oder Moleküle, die aufgrund einer Reaktionsraumverengung zu einer Beschleunigung der Hybridisierungsreaktion führen können (Polyethylenglycol, Polyvinylpyrrolidon, Dextransulfat u.ä.). Solche Zusätze werden vom Fachmann in den bekannten und üblichen Konzentrationen dem Hybridisierungspuffer zuzugeben.

Es versteht sich, dass der Fachmann die angegebenen Konzentrationen der Bestandteile des Hybridisierungspuffer derart auswählen kann, dass die gewünschte Stringenz der Hybridisierungsreaktion erzielt wird. Besonders bevorzugte Ausführungsformen geben stringente bis besonders stringente Hybrisidierungsbedingungen wieder. Unter Einsatz dieser stringenten Bedingungen kann der Fachmann feststellen, ob ein bestimmtes Nukleinsäuremolekül einen spezifischen Nachweis von Nukleinsäuresequenzen von Naeglerienarten ermöglicht und somit im Rahmen der Erfindung zuverlässig eingesetzt werden kann.

Die Konzentration der Sonde, kann je nach Markierung und Anzahl der zu erwartenden Zielstruktur stark schwanken. Um eine schnelle und effiziente Hybridisierung zu ermöglichen, sollte die Sondenmenge die Anzahl der Zielstrukturen um mehrere Größenordnungen überschreiten. Allerdings ist bei der Fluoreszenz in situ-Hybridisierung (FISH) darauf zu achten, dass eine zu hohe Menge an fluoreszenzmarkierter Hybridisierungssonde zu erhöhter Hintergrundfluoreszenz führt. Die Menge an Sonde sollte deshalb in einem Bereich zwischen 0,5 ng/µl und 500 ng/µl, bevorzugt zwischen 1,0 ng/µl und 100 ng/µl und besonders bevorzugt bei 50 ng/µl liegen.

Auf die Hybridisierung folgt ein stringenter Waschschritt, der ein Ablösen eventuell unspezifisch gebundener Sondenmoleküle bewirken soll. Hierbei kommen Pufferlösungen zum Einsatz, die prinzipiell sehr ähnlich aussehen können, wie Hybridisierungspuffer (gepufferte Natriumchloridlösung), nur dass der Waschschritt in einem Puffer mit niedrigerer Salzkonzentration, bzw. bei höherer Temperatur durchgeführt wird.

Zur theoretischen Abschätzung der Hybridisierungsbedingungen kann folgende Formel verwendet werden:

$$Td = 81,5 + 16,6 \lg[Na+] + 0,4 \text{ x } (\% \text{ GC}) - 820/n - 0,5 \text{ X } (\% \text{ FA})$$

Td = Dissoziationstemperatur in °C
[Na+] = Molarität der Natriumionen
% GC = Anteil der Guanin- und Cytosinnukleotide an der Anzahl der Basen
n = Länge des Hybrids
% FA= Formamidgehalt

Mit Hilfe dieser Formel kann z.B. der Formamidanteil (der wegen der Toxizität des Formamids möglichst gering sein sollte) des Waschpuffers ersetzt werden durch einen entsprechend niedrigeren Natriumchloridgehalt.

In der bevorzugten Ausführung dieser Erfindung beträgt der Natriumchloridgehalt des Waschpuffers 0,014 mol/l bis 0,9 mol/l, besonders bevorzugt 0,225 mol/l, bei 0,02 mol/l Tris-HCl, pH 8,0 und 0,01 % SDS, sowie bei 0 - 0,005 mol/l EDTA, besonders bevorzugt 0 mol/l EDTA.

Allerdings ist dem Fachmann aus der umfangreichen Literatur zu in situ-Hybridisierungsmethoden bekannt, dass und auf welche Weise die genannten Bestandteile variiert werden können.

Bezüglich der Stringenz der Hybrdisierungsbedingungen gilt das oben im Zusammenhang mit dem Hybridisierungspuffer Gesagte.

In einer alternativen Ausführungsform des erfindungsgemäßen Verfahrens werden die erfindungsgemäßen Nukleinsäuresondenmoleküle im sogenannten Fast-FISH-Verfahren zum spezifischen Nachweis von *Naeglaria*-Arten eingesetzt. Das Fast-FISH-Verfahren ist dem Fachmann bekannt und z.B. in der deutschen Patentanmeldung DE 199 36 875.9 und der internationalen Anmeldung WO 99/18234 beschrieben. Auf die in diesen Dokumenten enthaltene Offenbarung zur Durchführung der dort beschriebenen Nachweisverfahren wird hiermit ausdrücklich Bezug genommen.

Ein wichtiger Vorteil des in dieser Anmeldung beschriebenen Verfahrens zum spezifischen Nachweis von *Naeglaria*-Arten gegenüber herkömmlichen Nachweismethoden ist seine Schnelligkeit. Da der Tod durch eine *Naegleria fowleri*-Infektion innerhalb weniger Tage eintritt, ist der schnelle und vor allem spezifische Nachweis unbedingt notwendig, um noch rechtzeitig geeignete Therapeutika (z.B. Amphotericin B) anwenden zu können. Bis heute findet aber aufgrund der Langsamkeit herkömmlicher Verfahren eine Diagnose meist postmortem bei einer Gehirnautopsie statt.

Ein weiterer Vorteil liegt in der Spezifität dieses Verfahrens. Durch die verwendeten Gensonden können spezifisch sämtliche Arten der Gattung *Naegleria,* aber auch hochspezifisch nur die pathogene Art *Naegleria fowleri* nachgewiesen und visualisiert werden. Durch die Visualisierung der Naeglerien kann eine gleichzeitige visuelle Kontrolle stattfinden.

Ein weiterer Vorteil dieser Methode liegt auch in dem möglichen Verzicht auf Kultivierung.

Durch das Verfahren können leicht große Mengen an Proben auf das Vorhandensein auf Naeglerienzellen und im besondern auf das Vorhandensein von *Naegleria fowleri* getestet werden.

Die Vielzahl möglicher Markierungen ermöglich auch den gleichzeitigen Nachweis von zwei oder mehreren sich überlappenden oder auch nicht überlappenden Populationen. So kann z.B. durch die Verwendung von zwei verschiedenen Fluoreszenzmarkern neben allen Zellen, die der Gattung *Naegleria* zugehörig sind, auch spezifisch *Naegleria fowleri* nachgewiesen werden.

Das erfindungsgemäße Verfahren kann vielfältig angewendet werden. Umweltproben können auf das Vorhandensein von Naeglerien untersucht werden. Diese Proben können hierzu aus Luft, Wasser oder aus dem Boden entnommen sein.

Ein weiteres Anwendungsgebiet für das erfindungsgemäße Verfahren ist die Untersuchung von Lebensmitteln. Hierzu gehören vor allem mit Wasser vermengte Lebensmittel.

Das erfindungsgemäße Verfahren kann weiter zur Untersuchung medizinischer Proben eingesetzt werden. Es ist für die Untersuchung von Gewebeproben, z.B. Biopsiematerial aus dem Gehirn, der Lunge, Tumor- oder entzündliches Gewebe, aus Sekreten wie Schweiß, Speichel, Sperma und Ausfluss aus der Nase, Harnröhre oder Vagina sowie für Urin- oder Stuhlproben geeignet.

Ein weiteres Anwendungsgebiet für das vorliegende Verfahren ist die Untersuchung von Gewässern, z.B.

Badegewässer.

Erfindungsgemäß wird weiterhin ein Kit zur Durchführung des Verfahrens zum schnellen und hochspezifischen Nachweis von Naeglerienzellen in einer Probe zur Verfügung gestellt. Der Kit umfaßt als wichtigsten Bestandteil eine für den jeweils nachzuweisenden Mikroorganismus spezifische Oligonukleotidsonde. Weiterhin umfaßt er einen Hybridisierungs- und einen Waschpuffer. Die Wahl des Hybridisierungspuffers hängt in erster Linie von der Länge der verwendeten Nukleinsäuresonden ab. Beispiele für Hybridisierungsbedingungen sind in Stahl & Amann (1991, in Stackebrandt u. Goodfellow (Hrsg.), Nucleic Acid Techniques in Bacterial Systematics; John Wiley & Sons Ltd., Chichester, UK) angegeben. Der Kit enthält mindestens eine der oben genannten spezifischen Sonden zum Nachweis von Naeglerien, vorzugsweise enthält er mindestens eine Sonde, die für den Nachweis aller Arten der Gattung *Naegleria* geeignet ist, also bevorzugt NAEG1 oder NAEG2, und mindestens eine Sonde, die für den spezifischen Nachweis der Art *Naegleria fowleri* geeignet ist, also NFOW1 oder NFOW2.

Das folgende Beispiel soll die Erfindung erläutern, ohne sie einzuschränken:

Beispiel

Nachweis von Naeglerien in einer Wasserprobe

Eine Wasserprobe wird zentrifugiert und das Pellet mit 1/10 Volumen von mind. 37 % Paraformaldehyd (Merck, Darmstadt, Deutschland) vermengt und gut gemischt. Die Suspension wird für 5 Minuten bei Raumtemperatur inkubiert. Anschließend werden die Zellen bei 1.300 g für 5 Minuten zentrifugiert, der Überstand verworfen und das Pellet mit einem geeigneten Volumen 1 x PBS ($Na_xPO_4$) aufgenommen. Hier sind die Volumina frei wählbar, wobei allerdings Volumina bevorzugt werden, die noch gut in ein Eppendorf-Reaktionsgefäß passen und noch gut zentrifugierbar sind, wie z.B. 100-500 $\mu$l. Nach vollständiger Resuspension des Pellets wird das selbe Volumen an absolutem Ethanol zugegeben. In dieser Form sind die Naeglerien bei - 20 °C für mindestens 3 Monate lagerbar.

Zur Durchführung der Hybridisierung wird ein geeignetes Aliquot der fixierten Zellen (also z.B. 8-10 $\mu$l) auf einen Objektträger aufgebracht. Dazu können die Naeglerienzellen entweder einzeln oder gemischt mit anderen Naglerienspezies oder Acanthamöbenspezies oder Bakterienspezies gemischt werden.

Die Hybridisierung der Naeglerien erfolgt ohne die sonst nach dem Stand der Technik übliche aufsteigende Ethanolreihe zur Permeabilisierung der Zellmembranen.

Die Hybridisierung wird mit den oben angegebenen Sonden NAEG1 oder NAEG2 zur Detektion von Amöben der Gattung *Naegleria* (*N. fowleri, N. gruberi, N. clarki, N. australiensis, N. lovanensis, N. jamiesoni, N. italica, N. andersoni,* und *N. minor*) oder mit den ebenfalls oben angegebenen Sonden NFOW1 oder NFOW2 zur Detektion der humanpathogenen Amöbe *N. fowleri* durchgeführt. Die Sonden werden in einer Konzentration von 5 ng/$\mu$l eingesetzt. Allgemein ist ein Konzentrationsbereich zwischen 1 und 100 ng/$\mu$l geeignet.

Auf einem Objektträger werden einige Mikroliter der fixierten Naeglerienzellen aufgetragen und 20 min getrocknet. Anschließend werden mehrere Mikroliter eines Hybridisierungspuffers (0,9 mol/l NaCl, 0,02 mol/l Tris/HCl, 0,01 % SDS, 20 % Formamid) auf das Well aufgetragen und anschließend für 90 Minuten bei 46°C in einer feuchten Kammer inkubiert. Anschließend werden die Objektträger aus der Kammer entnommen kurz mit Waschpuffer (0,225 mol/l NaCl, 0,02 mol/l Tris, 0,01 % SDS) gespült und in diesem Puffer bei 48°C für 15 Minuten stringent gewaschen. Stringent bedeutet hier, dass die Waschbedingungen, wie oben im Detail beschrieben, so gewählt sind, dass die Zielnukleinsäure noch erfasst wird, während die nächst verwandten Nicht-Zielnukleinsäuren nicht erfasst werden, also abgewaschen werden.

Die Objektträger werden dann mit destilliertem Wasser abgespült und luftgetrocknet.

Optional kann zusätzlich noch eine unspezifische Nukleinsäurefärbung mit dem Farbstoff (DAPI, 4',6-Diamidino-2-phenylindol-dihydrochlorid; Sigma; Deisenhofen, Deutschland) durchgeführt werden. Dazu werden die Proben mit einer PBS-Lösung überschichtet, die 1 $\mu$g/ml DAPI enthält, und 5 -15 Minuten im Dunklen bei Raumtemperatur inkubiert. Nach einem weiteren Waschschritt mit destilliertem Wasser können die Proben dann in einem geeigneten Einbettmedium (Citifluor AF1, Citifluor Ltd., London, Großbritannien; Vectashild, Vector laboratories, Burlingame, USA) mit Hilfe eines Fluoreszenzmikroskops analysiert werden.

**Patentansprüche**

1. Oligonukleotid, das eine Nukleotidsequenz, ausgewählt aus der Gruppe, bestehend aus
   5'-ACC-ATA-GCG-CTC-GCT-GGT-3',
   5'-GTG-GCC-CAC-GAC-AGC-TTT-3' ,
   5'-GGT-CGA-TGC-CCA-GCT-CCC-3' und
   5'-GTC-AAA-GCC-TTG-TTT-GTC-3',
   aufweist.

2. Verfahren zum Nachweis von Protozoen der Gattung *Naegleria* in einer Probe, umfassend die Schritte

   a) Fixieren der in der Probe enthaltenen *Naegleria*-Zellen
   b) Inkubieren der fixierten Zellen mit mindestens einem Oligonukleotid, ausgewählt aus der Gruppe, bestehend aus

   i) den Oligonukleotiden nach Anspruch 1,
   ii) Oligonukleotiden, die mit den Oligonukleotiden nach Anspruch 1 in mindestens 60 % übereinstimmen und eine spezifische Hybridisierung mit Nukleinsäuresequenzen von *Naeglaria*-Zellen ermöglichen,
   iii) Oligonukleotiden, die sich von den Oligonukleotiden nach Anspruch 1 durch eine Deletion und/oder Addition unterscheiden und eine spezifische Hybridisierung mit Nukleinsäuresequenzen von *Naeglaria*-Zellen ermöglichen, und
   iv) Oligonukleotiden, die mit den vorgenannten Oligonukleotiden unter stringenten Bedingungen hybridisieren,

   um eine Hybridiserung herbeizuführen,
   c) Entfernen nicht hybridisierter Oligonukleotide,
   d) Detektieren und, gegebenenfalls Quantifizieren und Visualisieren, der *Naegleria*-Zellen mit hybridisierten Oligonukleotiden.

3. Verfahren nach Anspruch 2, wobei das Oligonukleotid mit einem detektierbaren Marker, ausgewählt aus der Gruppe bestehend aus

   a) Fluoreszenzmarker,
   b) Chemolumineszenzmarker,
   c) radioaktive marker,
   d) enzymatisch aktive Gruppen,
   e) Hapten
   f) durch Hybridisierung nachweisbare Nukleinsäuren,

   kovalent verbunden ist.

4. Verfahren nach Anspruch 2 oder 3, wobei die Probe eine Umweltprobe und aus Wasser, Boden oder Luft entnommen ist.

5. Verfahren nach Anspruch 2 oder 3, wobei die Probe eine Lebensmittelprobe ist.

6. Verfahren nach Anspruch 2 oder 3, wobei die Probe eine medizinische Probe ist.

7. Verfahren nach einem der Ansprüche 2 bis 6, wobei die Detektion mittels Epifluoreszenzmikroskopie erfolgt.

8. Verfahren nach einem der Ansprüche 2 bis 6, wobei die Detektion mittels Durchflußzytometrie erfolgt.

9. Verfahren nach einem der Ansprüche 2 bis 8, wobei es sich bei den *Naeglaria*-Zellen um Zellen der Art *Naeglaria fowleri* handelt.

10. Verwendung eines Oligonukleotids, ausgewählt aus der Gruppe, bestehend aus

    i) den Oligonukleotiden nach Anspruch 1,

ü) Oligonukleotiden, die mit den Oligonukleotiden nach Anspruch 1 in mindestens 60 % übereinstimmen und eine spezifische Hybridisierung mit Nukleinsäuresequenzen von *Naeglaria*-Zellen ermöglichen,

iii) Oligonukleotiden, die sich von den Oligonukleotiden nach Anspruch 1 durch eine Deletion und/oder Addition unterscheiden und eine spezifische Hybridisierung mit Nukleinsäuresequenzen von *Naeglaria*-Zellen ermöglichen, und

iv) Oligonukleotiden, die mit den vorgenannten Oligonukleotiden unter stringenten Bedingungen hybridisieren, als Hybridisierungssonde zum Nachweis von *Naeglaria*-Zellen in einer Probe.

**11.** Kit zur Durchführung des Verfahren nach einem der Ansprüche 2 bis 9, enthaltend mindestens ein Oligonukleotid, ausgewählt aus der Gruppe, bestehend aus

i) den Oligonukleotiden nach Anspruch 1,

ii) Oligonukleotiden, die mit den Oligonukleotiden nach Anspruch 1 in mindestens 60 % übereinstimmen und eine spezifische Hybridisierung mit Nukleinsäuresequenzen von *Naeglaria*-Zellen ermöglichen,

iii) Oligonukleotiden, die sich von den Oligonukleotiden nach Anspruch 1 durch eine Deletion und/oder Addition unterscheiden und eine spezifische Hybridisierung mit Nukleinsäuresequenzen von *Naeglaria*-Zellen ermöglichen, und

iv) Oligonukleotiden, die mit den vorgenannten Oligonukleotiden unter stringenten Bedingungen hybridisieren.

**12.** Kit nach Anspruch 11, weiter enthaltend eine Hybridisierungslösung und eine Waschlösung.


**Claims**

**1.** Oligonucleotide which has a nucleotide sequence selected from the group consisting of
5'-ACC-ATA-GCG-CTC-GCT-GGT-3',
5'-GTG-GCC-CAC-GAC-AGC-TTT-3',
5'-GGT-CGA-TGC-CCA-GCT-CCC-3' and
5'-GTC-AAA-GCC-TTG-TTT-GTC-3'.

**2.** Method for the detection of protozoa of the genus *Naegleria* in a sample, comprising the steps

a) fixing the *Naegleria* cells contained in the sample

b) incubating the fixed cells with at least one oligonucleotide

selected from the group consisting of

i) the oligonucleotides according to claim 1,

ii) oligonucleotides which are an at least 60% match with the oligonucleotides according to claim 1 and facilitate a specific hybridisation with nucleic acid sequences of *Naeglaria* cells,

iii) oligonucleotides which differ from the oligonucleotides according to claim 1 by a deletion and/or addition and facilitate a specific hybridisation with nucleic acid sequences of *Naeglaria* cells, and

iv) oligonucleotides which hybridise with the above-mentioned oligonucleotides under stringent conditions,

to produce a hybridisation,

c) removing non-hybridised oligonucleotides,

d) detecting and optionally quantifying and visualising the *Naegleria* cells with hybridised oligonucleotides.

**3.** Method according to claim 2, wherein the oligonucleotide is covalently connected to a detectable marker selected from the group consisting of

a) fluorescence markers,

b) chemiluminiscence markers,

c) radioactive markers,

d) enzymatically active groups,

e) hapten

f) nucleic acids detectable by hybridisation.

**4.** Method according to claim 2 or 3, wherein the sample is an environmental sample and is taken from water, soil or air.

**5.** Method according to claim 2 or 3, wherein the sample is a food sample.

**6.** Method according to claim 2 or 3, wherein the sample is a medical sample.

**7.** Method according to one of claims 2 to 6, wherein detection is carried out by means of epifluorescence microscopy.

**8.** Method according to one of claims 2 to 6, wherein detection is carried out by means of throughflow cytometry.

**9.** Method according to one of claims 2 to 8, wherein the *Naeglaria* cells are cells of the *Naeglaria fowleri* species.

**10.** Use of an oligonucleotide selected from the group consisting of

> v) the oligonucleotides according to claim 1,
> vi) oligonucleotides which are an at least 60% match with the oligonucleotides according to claim 1 and facilitate a specific hybridisation with nucleic acid sequences of *Naeglaria* cells,
> vii) oligonucleotides which differ from the oligonucleotides according to claim 1 by a deletion and/or addition and facilitate a specific hybridisation with nucleic acid sequences of *Naeglaria* cells, and
> viii) oligonucleotides which hybridise with the above-mentioned oligonucleotides under stringent conditions,

as hybridisation probe for the detection of *Naeglaria* cells in a sample.

**11.** Kit for carrying out the method according to one of claims 2 to 9, containing at least one olignucleotide selected from the group consisting of

> i) the oligonucleotides according to claim 1,
> ii) oligonucleotides which are an at least 60% match with the oligonucleotides according to claim 1 and facilitate a specific hybridisation with nucleic acid sequences of *Naeglaria* cells,
> iii) oligonucleotides which differ from the oligonucleotides according to claim 1 by a deletion and/or addition and facilitate a specific hybridisation with nucleic acid sequences of *Naeglaria* cells, and
> iv) oligonucleotides which hybridise with the above-mentioned oligonucleotides under stringent conditions.

**12.** Kit according to claim 11, further containing a hybridisation solution and a wash solution.

**Revendications**

**1.** Oligonucléotide présentant une séquence nucléotidique choisie dans le groupe constitué par
5'-ACC-ATA-GCG-CTC-GCT-GGT-3',
5'-GTG-GCC-CAC-GAC-AGC-TTT-3',
5'-GGT-CGA-TGC-CCA-GCT-CCC-3' et
5'-GTC-AAA-GCC-TTG-TTT-GTC-3'.

**2.** Procédé de détermination de protozoaires du genre *Naeglaria* dans un échantillon, comprenant les étapes consistant à :

> a) Fixer les cellules de *Naeglaria* contenues dans l'échantillon,
> b) Incuber les cellules fixées avec au moins un oligonucléotide choisi dans le groupe constitué par :

>> i) les oligonucléotides selon la revendication 1,
>> ii) des oligonucléotides qui présentent au moins 60% d'identité avec les oligonucléotides selon la revendication 1 et qui permettent une hybridation spécifique avec des séquences d'acides nucléiques de cellules de *Naeglaria,*
>> iii) des oligonucléotides qui se différencient des oligonucléotides selon la revendication 1 par une délétion et/ou une addition et qui permettent une hybridation spécifique avec des séquences d'acides nucléiques de cellules de *Naeglaria,* et
>> iv) des oligonucléotides qui s'hybrident en conditions stringentes avec les oligonucléotides précités, afin de provoquer une hybridation,

c) éliminer les oligonucléotides non hybridés

d) détecter, et le cas échéant quantifier et visualiser, les cellules de *Naeglaria* avec des oligonucléotides hybridés.

3. Procédé selon la revendication 2, dans lequel l'oligonucléotide est lié de manière covalente à un marqueur détectable choisi dans le groupe constitué par :

a) des marqueurs fluorescents,
b) des marqueurs chimioluminescents,
c) des marqueurs radioactifs,
d) des groupes enzymatiquement actifs,
e) des haptènes,
f) des acides nucléiques détectables par hybridation.

4. Procédé selon la revendication 2 ou 3, dans lequel l'échantillon est un échantillon environnemental et est prélevé dans l'eau, le sol ou l'air.

5. Procédé selon la revendication 2 ou 3, dans lequel l'échantillon est un échantillon alimentaire.

6. Procédé selon la revendication 2 ou 3, dans lequel l'échantillon est un échantillon médical.

7. Procédé selon l'une des revendications 2 à 6, dans lequel la détection se fait par microscopie à épifluorescence.

8. Procédé selon l'une des revendications 2 à 6, dans lequel la détection se fait par cytométrie de flux.

9. Procédé selon l'une des revendications 2 à 8, dans lequel les cellules de *Naeglaria* sont des cellules de l'espèce *Naeglaria fowleri.*

10. Utilisation d'un oligonucléotide choisi dans le groupe constitué par :

i) les oligonucléotides selon la revendication 1,
ii) des oligonucléotides qui présentent une identité d'au moins 60% avec les oligonucléotides selon la revendication 1 et qui permettent une hybridation spécifique avec des séquences d'acides nucléiques de cellules de *Naeglaria*,
iii) des oligonucléotides qui se différencient des oligonucléotides selon la revendication 1 par une délétion et/ou une addition et qui permettent une hybridation spécifique avec des séquences d'acides nucléiques de cellules de *Naeglaria*, et
iv) des oligonucléotides qui s'hybrident en conditions stringentes avec les oligonucléotides précités,

comme sonde d'hybridation pour la détermination de cellules de *Naeglaria* dans un échantillon.

11. Kit pour la mise en oeuvre du procédé selon l'une des revendications 2 à 9, contenant au moins un oligonucléotide choisi dans le groupe constitué par :

i) les oligonucléotides selon la revendication 1,
ii) des oligonucléotides qui présentent une identité d'au moins 60% avec les oligonucléotides selon la revendication 1 et qui permettent une hybridation spécifique avec des séquences d'acides nucléiques de cellules de *Naeglaria,*
iii) des oligonucléotides qui se différencient des oligonucléotides selon la revendication 1 par une délétion et/ou une addition et qui permettent une hybridation spécifique avec des séquences d'acides nucléiques de cellules de *Naeglaria,* et
iv) des oligonucléotides qui s'hybrident en conditions stringentes avec les oligonucléotides précités.

12. Kit selon la revendication 11, contenant en plus une solution d'hybridation et une solution de lavage.

SEQUENZPROTOKOLL

<110> Vermicon AG

<120> Verfahren zum Nachweis von Protozoen der Gattung Naegleria

<130> V 7299

<140> DE 100 57 841
<141> 2000-11-22

<160> 4

<170> PatentIn Ver. 2.1

<210> 1
<211> 18
<212> DNA
<213> Künstliche Sequenz

<220>
<223> Beschreibung der künstlichen Sequenz:
Oligonukleotidsonde

<400> 1
accatagcgc tcgctggt          18

<210> 2
<211> 18
<212> DNA
<213> Künstliche Sequenz

<220>
<223> Beschreibung der künstlichen Sequenz:
Oligonukleotidsonde

<400> 2
gtggcccacg acagcttt          18

<210> 3
<211> 18
<212> DNA
<213> Künstliche Sequenz

<220>
<223> Beschreibung der künstlichen Sequenz:
Oligonukleotidsonde

<400> 3
ggtcgatgcc cagctccc          18

<210> 4
<211> 18
<212> DNA
<213> Künstliche Sequenz

<220>

<223> Beschreibung der künstlichen Sequenz:
Oligonukleotidsonde

<400> 4
gtcaaagcct tgtttgtc          18